# FASCICULE DE BREVET EUROPEEN

(11) **EP 2 014 231 B1**
(45) Date de publication et mention de la délivrance du brevet: **16.11.2011**
(21) Numéro de dépôt: 08290543.1
(22) Date de dépôt: 12.06.2008
(51) Int. Cl.: A61B 5/15, G01T 1/161, A61B 6/00

(54) **Dispositif de comptage de particules élémentaires émises par un fluide dans un conduit**
Zählvorrichtung für die von einer Flüssigkeit in einer Leitung abgegebene Elementarteilchen
Device for counting elementary particles emitted by a fluid in a pipe

(30) Priorité: 19.06.2007 FR 0704348
(43) Date de publication de la demande: 14.01.2009
(60) Demande divisionnaire: 11172205.4
(73) Titulaire: Commissariat à l'Énergie Atomique et aux Énergies Alternatives, 75015 Paris (FR)
(72) Inventeur: Reymond, Jean-Marc, 78470 Saint-Rémy-lès-Chevreuse (FR); Kerhoas-Cavata, Sophie, 78125 Raizeux (FR); Mangeot, Philippe, 94270 Le Kremlin-Bicêtre (FR)
(74) Mandataire: Bolinches, Michel Jean-Marie

(56) Documents cités:
- WO-A-03/003923
- WO-A-2007/093913
- US-A1- 2006 085 020
- US-A1- 2007 083 160
- CONVERT L ET AL: "A Microvolumetric /spl beta/ Blood Counter for Pharmacokinetic Pet Studies in Small Animals", NUCLEAR SCIENCE SYMPOSIUM CONFERENCE RECORD, 2005 IEEE WYNDHAM EL CONQUISTADOR RESORT, PUERTO RICO OCTOBER 23 - 29, 2005, PISCATAWAY, NJ, USA,IEEE, vol. 5, 23 October 2005 (2005-10-23), pages 2674-2678, XP010896217, DOI: DOI:10.1109/NSSMIC.2005.1596887 ISBN: 978-0-7803-9221-2

## Description

La présente invention concerne un dispositif de comptage de particules élémentaires (telles que des particules α, γ, des électrons ou des positons) émises par un fluide, le dispositif comportant un conduit de transfert de ce fluide et, à l'extérieur du conduit, des moyens de détection de ces particules qui sont atténuées par une paroi du conduit et/ou par ce fluide. L'invention s'applique plus particulièrement, mais non exclusivement, à un fluide constitué de microéchantillons sanguins d'un mammifère.

De manière connue, le comptage de particules atténuées par certains composants d'un fluide les incorporant et/ou de la paroi du conduit transférant ce fluide pose certains problèmes techniques, qui sont particulièrement pénalisants dans le cas particulier du comptage au sein de petits échantillons de fluides, dans le cas d'absorption de ces particules par le fluide, ou bien lorsque les particules à compter sont des électrons ou des positons émis par radioactivité bêta, notamment. Parmi les applications cumulant ces trois problèmes, on peut principalement citer celles qui mesurent directement les positons ou les électrons issus de la radioactivité bêta contenue dans des microéchantillons sanguins, par exemple pour les dispositifs de mesure de la fonction d'entrée d'un mammifère destinés à l'imagerie quantitative.
Des laboratoires comme celui de Sherbrooke au Canada ont développé des détecteurs de positons ou d'électrons pour des microéchantillons sanguins incorporant des émetteurs bêta à titre de radiotraceurs, en proposant des dispositifs de comptage dont l'efficacité des détecteurs ne dépasse pas 7 % dans le cas de radiotraceurs au ¹⁸F et 16 % dans le cas de radiotraceurs au ¹¹C. Ces dispositifs comprennent un conduit tubulaire de section de passage constante sur toute sa longueur, sans particularité aucune dans sa zone de détection pourvue des détecteurs. Ce conduit présente un diamètre interne de 0,58 mm, et comme le libre parcours moyens des positons dans le sang est de l'ordre de 0,6 mm pour le ¹⁸F, il en résulte que peu de positons sortent de ces microéchantillons sanguins. De plus, le matériau utilisé pour ce conduit - un polyéthylène de basse densité (PEBD) - présentant une épaisseur de paroi e très élevée (e ≥ 150 µm et typiquement égale à 192,5 µm) pour une masse volumique d proche de 1 g/cm³ (typiquement égale à 0,92 g/cm³ pour du « PE10 »), il en résulte que ce conduit génère une atténuation des électrons ou des positons qui est relativement élevée, étant proportionnelle de manière connue au produit e.d qui est ici typiquement égal à 192,5 x 0,92 soit à environ 177.

Un dispositif de comptage selon le préambule de la revendication 1 et décrit dans l'article "A Microvolumetric β Blood Counter for Pharmacokinetic PET Studies in Small Animals" par L. Convert, IEEE Nuclear Science Symposium Conference Record.

Sont également connus sous la dénomination « β-microprobe » d'autres méthodes et dispositifs de comptage de particules qui sont utilisés par la société française Biospace pour la mesure de la fonction d'entrée d'un petit mammifère, tel qu'un rat ou une souris. Ces méthodes consistent essentiellement à éliminer le conduit précité en introduisant directement le détecteur dans une artère de l'animal, à l'état immergé dans le sang de ce dernier.

Un inconvénient majeur de cette solution sans conduit est que le radiotraceur accumulé dans les autres organes de l'animal crée une forte perturbation par rayonnements gamma. Un autre inconvénient de cette solution réside dans les faibles dimensions caractérisant la mesure, ce qui conduit à une faible efficacité de comptage. Il est alors nécessaire d'adjoindre au détecteur principal un second détecteur, que l'on place dans le corps de l'animal mais hors du sang de celui-ci et que l'on utilise de manière différentielle pour soustraire le bruit de fond dû à ces rayonnements gamma.

Même dans ces conditions de mesure, il s'avère que l'efficacité de détection (réduite à l'absorption dans le sang) annoncée est seulement de 16 % et de 20 % pour des radiotraceurs à base de ¹⁸F et de 11C, respectivement, avec un détecteur plastique de type scintillant de 1 mm de diamètre.

Etant donné l'évolution récente des technologies vers le traitement de microéchantillons et la volonté des biologistes de développer des outils destinés aux petits animaux, tels que des souris, il est fondamental de pouvoir mesurer l'activité de microéchantillons liquides contenant le moins possible de radiotraceurs. Ces microéchantillons présentant un volume de l'ordre de quelques µL (typiquement de 8 à 30 µL), il est nécessaire que les dispositifs de comptage utilisés présentent une efficacité (i.e. une sensibilité de mesure) élevée, particulièrement pour l'étude de radiotraceurs à vie courte comme le ¹¹C, dont l'activité en fin de séquence diminue de façon significative (la demi-vie du ¹¹C étant de 20 minutes).

Un but de la présente invention est de proposer un dispositif de comptage de particules élémentaires émises par un fluide, le dispositif comportant un conduit de transfert de ce fluide et, agencé à l'extérieur du conduit, des moyens de détection de ces particules, lesquelles sont atténuées par une paroi du conduit et/ou par ce fluide, dispositif qui remédie aux inconvénients précités en présentant une sensibilité de mesure améliorée.
A cet effet, un dispositif de comptage selon l'invention est tel qu'il comprend au moins un tronçon de comptage de section transversale oblongue qui relie entre elles deux portions adjacentes de ce conduit présentant une plus grande section de passage et qui présente un ratio [hauteur interne / largeur interne] inférieur ou égal à 20 %, où la hauteur et la largeur internes représentent respectivement la plus petite et la plus grande des dimensions transversales du tronçon mesurées selon deux directions sensiblement perpendiculaires, lesdits moyens de détection s'étendant transversalement à ce tronçon en regard de toute sa largeur interne et de part et d'autre de celle-ci.

On notera que dans le cas où les particules à compter sont des électrons ou des positons, cette géométrie extrêmement aplatie du tronçon de comptage combinée à sa largeur interne relativement élevée en regard de laquelle sont agencés en dépassement lesdits moyens de détection procure une efficacité de comptage très élevée, typiquement supérieure à 50 % tant pour des radiotraceurs à base de ¹⁸F que de ¹¹C, permettant ainsi des mesures même sur des microéchantillons de sang de volume inférieur à 10 µL (typiquement prélevés sur des souris) et pouvant contenir des radiotraceurs à vie courte (tels que le ¹¹C).

On notera en particulier que cet agencement desdits moyens de détection à la fois en regard et en dépassement de la largeur transversale du tronçon de comptage permet de maximiser l'acceptance géométrique de ces moyens de détection (i.e. de maximiser l'ensemble des directions de l'espace selon lesquelles une molécule de radiotraceur peut émettre une particule élémentaire dont le trajet impacte les moyens de détection, rendant possible sa « capture »), contribuant ainsi à améliorer cette efficacité de comptage.
Par « section de passage », on entend dans la présente description la section transversale interne du tronçon de comptage (de forme oblongue ou aplatie) et celle de chacune des deux portions adjacentes à ce tronçon (de préférence circulaire).

Lorsque le fluide est un liquide, ce tronçon de comptage présente préférentiellement un ratio [hauteur interne / largeur interne] compris entre 5 % et 10 %.
Avantageusement, le ratio de la section de passage du tronçon sur celle de chaque portion adjacente peut être inférieur ou égal à 35 % et, encore plus avantageusement, être inférieur à 25 %.
Egalement avantageusement, ladite hauteur interne du tronçon de comptage peut être inférieure à 20 % du diamètre interne de chacune des portions cylindriques adjacentes, et ladite largeur interne de ce tronçon peut être supérieure à 1,3 fois ce diamètre interne.
Selon un mode préférentiel de réalisation de l'invention, ce tronçon de comptage présente une section transversale sensiblement rectangulaire dont les grands côtés et/ou les petits côtés sont incurvés selon des courbures symétriques l'une de l'autre, de sorte que ce tronçon présente au moins en partie une face externe sensiblement convexe ou concave.

Selon une autre caractéristique préférentielle de l'invention, ledit conduit est adapté à la circulation de microéchantillons liquides, tels que des microéchantillons sanguins, ladite hauteur interne du tronçon de comptage étant comprise entre 100 µm et 250 µm et ladite largeur interne de ce tronçon étant supérieure à 1,3 mm, alors que la ou chaque portion cylindrique adjacente présente un diamètre compris entre 0,8 mm et 1,2 mm.

Par « microéchantillons », on entendra dans la présente description des échantillons liquides présentant chacun un volume inférieur à 100 µL et, de préférence, inférieur ou égal à 30 µL (i.e. typiquement des échantillons prélevés sur des petits animaux).
Encore plus préférentiellement, l'aire de la section de passage dudit tronçon de comptage est comprise entre 0,15 mm² et 0,25 mm², et ce tronçon peut alors présenter une longueur comprise entre 30 mm et 40 mm de sorte à pouvoir contenir un microéchantillon d'environ 8 µL en regard desdits moyens de détection.

Spécifiquement lorsque les particules à compter sont des électrons ou des positons issus de la radioactivité bêta émise par ledit fluide, ledit tronçon de comptage présente avantageusement une paroi d'épaisseur e, exprimée en µm, et de masse volumique d, exprimée en g/cm³, dont le produit e.d est inférieur à 100 et de préférence inférieur à 50, de telle sorte que l'atténuation par ce tronçon des particules à compter soit minimisée.
Selon une autre caractéristique avantageuse de l'invention qui concerne notamment de telles particules de type électrons ou positions, ledit tronçon de comptage est à base d'un polymère thermoformé de masse volumique inférieure ou égale à 1,5 g/cm³, de préférence un polyimide de dénomination « Kapton », et ce tronçon présente une paroi d'épaisseur inférieure à 50 µm et de préférence inférieure à 30 µm. De préférence, ce matériau est choisi biocompatible si le liquide mesuré est biologique et susceptible d'être réinjecté à un être vivant.

On notera que cette sélection d'un tel polyimide de type « Kapton » permet d'obtenir cette valeur très faible pour le produit e.d précité, en comparaison des valeurs usuelles de ce produit généralement comprises entre 150 et 200 pour les conduits connus réalisés en PEBD, qui présentent une densité inférieure à celle de ce polyimide « Kapton » mais une épaisseur très nettement supérieure.

Selon une autre caractéristique de l'invention, lesdits moyens de détection comportent avantageusement deux ensembles de détecteurs respectivement disposés contre ou à proximité immédiate de deux grandes faces sensiblement planes dudit tronçon de comptage qui sont distantes entre elles de ladite hauteur et qui sont reliées entre elles par deux petites faces de ce tronçon, lesquelles sont dépassées dans le sens de ladite largeur par ces ensembles de détecteurs afin de maximiser l'acceptance géométrique de ces derniers.
Selon un mode particulier de réalisation de l'invention, ledit dispositif peut être agencé en amont d'une pompe péristaltique pilotée par ordinateur pour l'aspiration par à-coups d'une quantité déterminée d'échantillons à prélever, lesdites grandes faces dudit tronçon de comptage présentant alors chacune en leur côté externe une forme qui est avantageusement de type légèrement convexe.
On notera que cette géométrie convexe des grandes faces externes du tronçon de comptage est avantageusement utilisable de manière générale lorsque le fluide circulant dans le conduit est soumis à une surpression ou à une pression qui varie dans le temps, par exemple en cas d'aspirations successives des échantillons prélevés.

Est aussi décrite une méthode de comptage de particules élémentaires mise en oeuvre au moyen d'un dispositif de comptage tel que défini ci-dessus, et cette méthode consiste notamment à choisir pour la paroi dudit tronçon de comptage un matériau qui est sensiblement transparent aux particules émises par ledit fluide.
Avantageusement, ces particules sont des photons émis par fluorescence, et dans ce cas la paroi de ce tronçon doit être choisie transparente à la longueur d'onde des photons considérés.
Ces particules peuvent aussi être des électrons ou des positons émis par radioactivité bêta, ladite paroi présentant une épaisseur e, exprimée en µm, et une masse volumique d, exprimée en g/cm³, telle que le produit e.d soit inférieur à 100 et de préférence inférieur à 50, de sorte à minimiser l'absorption de ces particules afin de ne pas affecter significativement le comptage.

Avantageusement, cette méthode de comptage est telle que ledit fluide est constitué de microéchantillons sanguins qui circulent dans ledit conduit et dans le plasma desquels est dilué un radiotraceur bêta à vie courte, tel que le carbone 11.
Encore plus avantageusement selon cette méthode , ces microéchantillons ont été successivement prélevés dans un mammifère, tel qu'un rat ou une souris, et présentent chacun un volume inférieur à 100 µL et de préférence inférieur ou égal à 30 µL, la totalité du volume de chaque microéchantillon étant sensiblement localisée dans ledit tronçon de comptage en regard desdits moyens de détection, pour que ces microéchantillons se succèdent dans ce tronçon de manière discrétisée dans l'espace et dans le temps.
On notera que la petite taille des animaux qui sont de préférence utilisés pour la mesure des microéchantillons prélevés requiert de limiter le volume total de ces microéchantillons à une quantité qui est compatible non seulement avec la santé de l'animal, mais encore avec une perturbation aussi faible que possible de son métabolisme.

Les caractéristiques précitées de la présente invention, ainsi que d'autres, seront mieux comprises à la lecture de la description suivante de plusieurs exemples de réalisation de l'invention, donnés à titre illustratif et non limitatif, ladite description étant réalisée en relation avec les dessins joints, parmi lesquels :
la figure 1 est une vue schématique partielle d'un système automatisé de prélèvement et de mesure selon l'invention incluant un dispositif de comptage de radioactivité par exemple bêta, agencé en amont d'un dispositif d'échantillonnage des microéchantillons prélevés,
la figure 2 est une photographie illustrant la forme et les dimensions relatives, en comparaison d'une pièce de 10 centimes d'euro, d'un conduit de ce système de prélèvement et de mesure incluant un tronçon aplati apte à optimiser ce comptage de radioactivité,
la figure 3 est une vue schématique en section transversale de ce tronçon aplati selon l'invention équipé des deux ensembles de détecteurs illustrés à la figure 1, et
la figure 4 est un graphique illustrant l'efficacité de détection en radiotraceur ¹⁸F en fonction du seuil de détection, pour trois types de conduits de prélèvement comprenant ce tronçon de comptage aplati selon l'invention et, à titre d'essais comparatifs, deux micro-tubes cylindriques.

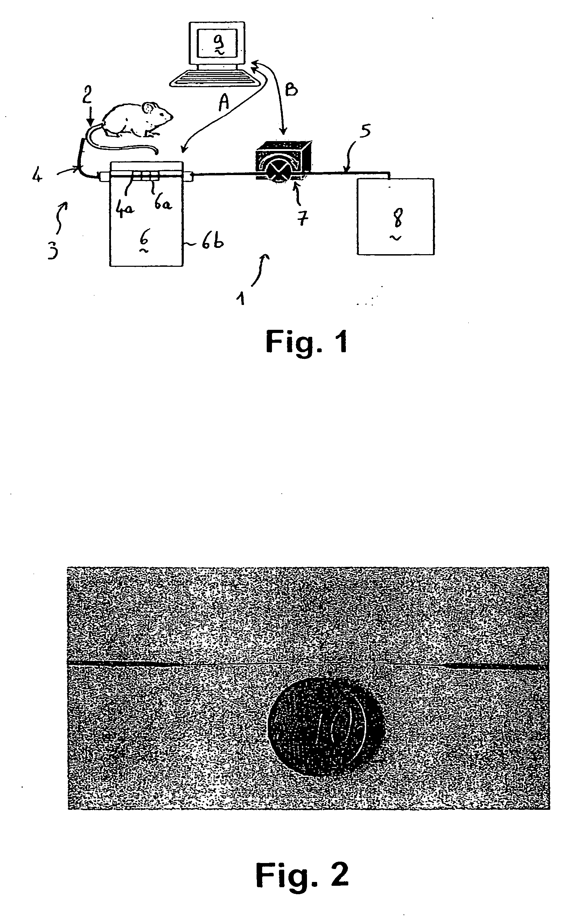

La figure 1 illustre à titre d'exemple une installation 1 automatisée pour procéder successivement et en continu au prélèvement, au stockage temporaire et à des mesures radiatives de microéchantillons sanguins d'un petit mammifère 2 par exemple de type rat ou souris, au moyen d'un système de prélèvement 3 incluant une succession de conduits 4 et 5 de type micro-tubes ou capillaires souples. Ce système de prélèvement 3 comporte essentiellement :
- un cathéter équipé d'un dispositif de raccordement (non illustrés) et qui est destiné à aspirer par à-coups une même quantité de sang à prélever, via une pompe péristaltique 7,
- un dispositif de comptage 6 de particules présentes dans les microéchantillons prélevés, qui est dans cet exemple un compteur de positons ou d'électrons issus de la radioactivité bêta 6 pour des microéchantillons de sang total et qui est placé au plus près du point de prélèvement en étant quasiment au contact d'un tronçon de comptage 4a de cette succession de conduits 4, 5 (comme développé ci-après, ce tronçon 4a présente des caractéristiques de forme et de matériau optimisées pour ce comptage et est centré par rapport aux diodes de détection 6a que comporte le dispositif 6),
- un système d'échantillonnage 8 agencé en aval de la pompe péristaltique 7, où ces microéchantillons prélevés et analysés sont stockés et traités, et
- un dispositif de commande 9 assisté par ordinateur pour la commande de l'ensemble du système 3, en incluant cette pompe 7 (voir les doubles flèches A et B à la figure 1 pour cette commande).
Selon l'invention, la succession de conduits 4 et 5 est avantageusement telle que les élargissements de section transversale présents sur ces conduits sont tous inférieurs ou égaux à 20 % en termes de ratios de surfaces, de sorte que les microéchantillons se suivant dans cette succession de conduits 4 et 5 ne s'y mélangent pratiquement pas entre eux par diffusion. De cette manière, ces microéchantillons prélevés sont discrétisés dans l'espace et dans le temps.

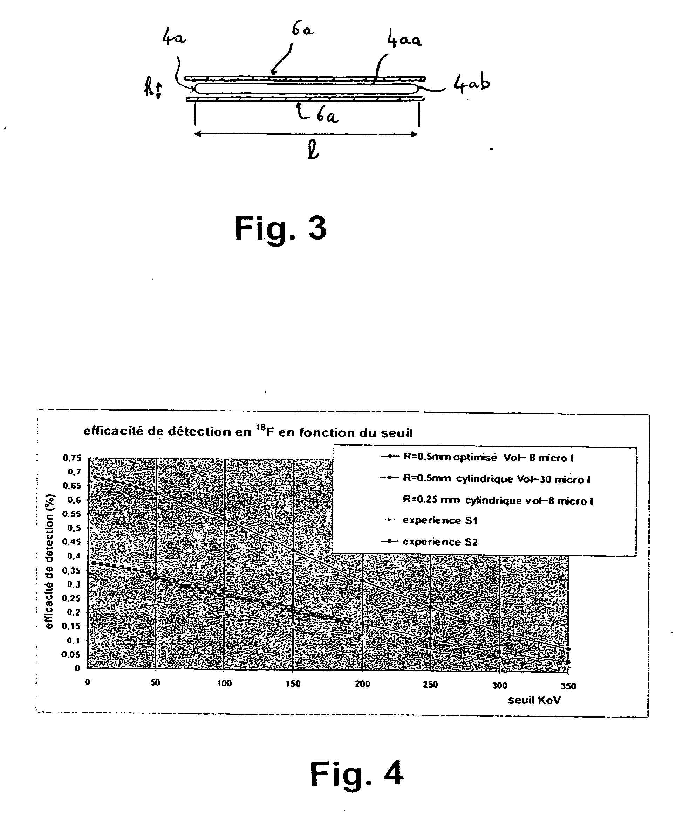

Comme illustré aux figures 2 et 3, on a prévu dans la succession de conduits de prélèvement 4, 5 selon l'invention un tronçon de comptage aplati 4a qui relie entre elles deux portions cylindriques et qui est conçu pour optimiser le comptage de particules par le dispositif 6 de la figure 1 (tel qu'un compteur bêta, que l'on utilise avantageusement pour la mesure de la fonction d'entrée du petit mammifère 2). On a ainsi choisi de réduire le volume de détection et d'augmenter l'efficacité de comptage.
A cet effet, on a façonné par thermoformage ce tronçon aplati 4a de section transversale oblongue qui est réalisé de préférence en un polyimide de dénomination « Kapton » (de masse volumique égale à 1,42 g/cm³, avec une paroi de 25 µm ±10 % d'épaisseur) et qui relie entre eux deux micro-tubes cylindriques par exemple en PEBD (polyéthylène de basse densité) de diamètre interne égal à 1 mm environ. Comme illustré à la figure 3, les diodes de détection 6a du dispositif 6 de comptage sont agencées de part et d'autre des faces externes des grands côtés 4aa du tronçon 4a, par rapport à sa plus petite dimension transversale constituée ici par sa hauteur h.
Dans cet exemple de réalisation, le tronçon aplati 4a présente une section transversale sensiblement rectangulaire dont les faces externes des petits côtés 4ab sont incurvées selon des courbures convexes symétriques l'une de l'autre, et ce tronçon présente un ratio [hauteur interne h / largeur interne I] d'environ 8 %, où la hauteur et la largeur internes sont respectivement égales à 130 µm et à 1490 µm.
Quant au ratio surfacique de la section de passage du tronçon aplati 4a - d'environ 0,1937 mm² - sur celle de chaque portion cylindrique adjacente (de section interne d'environ 0,785 mm²), il est légèrement inférieur à 25%.
En outre, le tronçon aplati 4a présente une paroi d'épaisseur e et de masse volumique d dont le produit e.d est sensiblement égal à 35,5 (avec e = 25 µm et d = 1,42 g/cm³), ce qui est très nettement inférieur aux valeurs usuellement utilisées qui sont généralement comprises entre 150 et 200 pour les micro-conduits réalisés en PEBD (qui présentent une densité inférieure à celle du « Kapton » mais une épaisseur nettement supérieure), de telle sorte que l'atténuation par ce tronçon 4a selon l'invention des particules à compter, telles que des électrons ou des positons issus de la radioactivité bêta, est nettement minimisée.
Comme illustré à la figure 3, le tronçon aplati 4a est équipé, en regard de ses grandes faces 4aa - qui sont de préférence légèrement convexes - et venant en dépassement de ses petites faces 4ab, de deux ensembles desdites diodes de détection 6a aptes à compter lesdites particules de chaque microéchantillon liquide y circulant (ce dépassement des diodes 6a permet d'optimiser leur acceptance géométrique, et donc la « capture » des particules à compter).
Le procédé de thermoformage utilisé pour l'obtention de ce tronçon aplati 4a selon l'invention comprend notamment les étapes suivantes :
- mise en place du tronçon 4a à froid dans le gabarit de formage,
- raccordement de ses deux extrémités à des micro-tubes souples pour la mise en pression,
- mise sous pression (1,5 bars de pression relative),
- chauffage du gabarit à 300° C pendant 15 minutes,
- refroidissement sous pression, et
- chute lente de la pression après refroidissement.
On obtient par ce thermoformage un tronçon aplati de comptage 4a dont les deux grandes faces 4aa présentent chacune une superficie d'environ 1,5 mm x 35 mm soit 52,5 mm², ce qui se traduit par une minimisation de l'épaisseur de liquide que le positon ou l'électron doit traverser pour atteindre les détecteurs 6a.

Le graphique de la figure 4 illustre, sous forme de courbes de simulation confirmées par l'expérience, les résultats d'efficacité de comptage obtenus pour deux séries d'expériences S1 et S2, réalisées chacune :
- avec un conduit selon l'invention de rayon égal à 0,5 mm pour les portions cylindriques et incorporant ce tronçon 4a aplati, avec un volume de microéchantillon sanguin prélevé de 8 µL (compatible avec une souris),
- avec un premier conduit « témoin » cylindrique sur toute sa longueur (i.e. sans tronçon aplati) présentant un rayon de 0,5 mm, avec un volume de microéchantillon sanguin prélevé de 30 µL (compatible avec un rat), et
- avec un second conduit « témoin » cylindrique sur toute sa longueur (i.e. sans tronçon aplati) présentant un rayon de 0,25 mm, avec un volume de microéchantillon sanguin prélevé de 8 µL.
Grâce à ce tronçon aplati 4a, on note que l'efficacité de détection des positons augmente, passant de 32 % avec les micro-tubes cylindriques à plus de 60 % avec le micro-tube de l'invention, pour le seuil minimum (d'environ 46 Kev). Le gain est même plus important car un conduit micro-tubulaire cylindrique compatible avec 8 µL donnerait environ 25 % d'efficacité. Le micro-tube optimisé selon l'invention permet ainsi de travailler avec des échantillons de 8 µL, en atteignant plus de 60 % d'efficacité au seuil minimum, contre seulement 25 % avec un micro-tube entièrement cylindrique sur sa longueur, ce qui rend le système de prélèvement 3 selon l'invention particulièrement bien adapté à la mesure de la fonction d'entrée d'une souris.

En relation avec la figure 1, on va à présent décrire plus précisément le dispositif et la méthode de comptage des particules bêta utilisés en relation avec le système de prélèvement automatisé 3.
Quelques centimètres en aval du premier conduit 4 de prélèvement, chaque microéchantillon passe au plus près du compteur de particules bêta 6, pour lequel l'épaisseur de la paroi du conduit n'apporte qu'une très faible atténuation. Le tronçon aplati 4a, fixé dans le boîtier 6b du compteur 6, permet de minimiser le taux d'annihilation des positons dans les parois, et sa géométrie est telle qu'il peut contenir le volume d'un échantillon (de 30 µL ou de 8 µL) correctement centré sous les six diodes de détection en silicium (10 x 10 x 0,3 mm³) entourant le tronçon 4a, comme illustré à la figure 1. Ces diodes 6a sont elles-mêmes entourées d'un blindage de plomb de 2 cm d'épaisseur destiné à supprimer le bruit physique venant des photons issus de l'animal 2. Le reste de ce système de mesure 6 comporte une carte électronique de traitement et interfaçage, l'ensemble privilégiant la compacité et la robustesse en formant un boîtier 6b de petites dimensions (8 x 10 x 4 cm³).
Il est avantageux, pour minimiser la probabilité d'annihilation dans le sang d'un positon issu de la radioactivité bêta, de donner au micro-tube souple la forme aplatie du tronçon 4a, au moins à l'endroit de son passage devant les diodes 6a. Par ailleurs, comme expliqué précédemment, cette configuration géométrique assure un étalement du liquide en une nappe fine, ce qui augmente la surface de liquide en regard des surfaces détectrices. La configuration retenue pour le système de mesure 6 est la suivante.
Le tronçon aplati 4a, de 25 µm d'épaisseur de paroi, est placé en « sandwich » entre les diodes 6a de 0,3 mm d'épaisseur (trois diodes 6a en haut et trois autres en bas).
L'électronique de lecture de ces diodes 6a ainsi que l'électronique gérant l'acquisition et le transfert de données ont été intégrées dans un unique module électronique, qui a été optimisé afin de réduire au maximum le bruit électronique permettant de minimiser le seuil de détection pour une meilleure efficacité.
L'électronique « front-end » (mise en forme et discriminateur) est assurée par un « ASIC » (comprenant 16 voies, un seuil commun, 16 sorties + 1 OR). Le seuil est réglable par l'utilisateur. La carte d'acquisition est une carte de test « USB » configurable qui tire parti de la souplesse de l'interface « USB » des ordinateurs personnels et des progrès des circuits numériques configurables « FPGA » (« Field Programmable Gate Array »). Elle permet de traiter rapidement un grand nombre de signaux et est programmable depuis l'interface d'un ordinateur.
Le schéma de principe est illustré à la figure 1. Le conduit de prélèvement 4 se prolonge par le tronçon aplati 4a qui prend le relais à l'intérieur du boîtier 6b, et le sang ressort de l'autre côté de celui-ci. Cette circulation du sang est réalisée grâce à la pompe péristaltique 7. Le volume des microéchantillons prélevés est réglable, ainsi que leur temps de prélèvement. Ces paramètres sont pilotés par l'ordinateur du dispositif de commande 9 de l'installation 1.
L'espacement minimum entre deux prélèvements de microéchantillons est de 1 seconde. Afin de couvrir la dynamique d'une cinétique de radiotraceur dans le sang, les microéchantillons sont prélevés toutes les secondes après l'injection pendant environ 30 secondes à 1 minute, puis ils sont prélevés de façon plus espacée dans le temps, la pente de la courbe étant plus faible pendant cette phase.
Les connexions entre les divers conduits et le boîtier 6b sont conçues pour éviter toute perte de volume des microéchantillons et toute diffusion entre deux microéchantillons adjacents n'est possible.
Ces microéchantillons sanguins sont ainsi prélevés de façon discrète dans l'espace et dans le temps, et ils progressent sans diffuser jusqu'au système d'échantillonnage 8 où ils sont stockés et avantageusement
soumis à l'extraction d'une au moins de leurs phases ou de leurs composants, par exemple mise en oeuvre par centrifugation.
On notera que ce système de prélèvement 3 selon l'invention peut être avantageusement utilisé dans le domaine de la recherche préclinique pour l'imagerie quantitative de nouveaux traceurs, notamment en tomographie par émission de positons (TEP). Dans ce cas, le liquide considéré est du sang, et l'application consiste à mesurer la fonction d'entrée pour de petits animaux comme les rats ou les souris. La faible taille de ces animaux, et donc la faible quantité totale de sang qu'ils possèdent, limitent le volume de chaque microéchantillon à environ 30 µL pour les rats, et à environ 8 µL pour les souris.
Une autre application possible de l'invention concerne un fluide gazeux constitué d'air marqué par exemple par un radio isotope du xénon. Dans ce cas le dispositif comprend un tronçon de comptage 4a de section transversale oblongue qui relie entre elles deux portions adjacentes de ce conduit présentant une plus grande section de passage et qui présente un ratio [hauteur interne (h) / largeur interne (I)] égal à 20 %, où la hauteur et la largeur internes représentent respectivement la plus petite et la plus grande des dimensions transversales de ce tronçon mesurées selon deux directions sensiblement perpendiculaires, lesdits moyens de détection s'étendant transversalement à ce tronçon en regard de toute sa largeur interne et de part et d'autre de celle-ci.

## Revendications

1. Dispositif de comptage (6) avec une sensibilité améliorée de particules élémentaires émises par un fluide, le dispositif comportant un conduit de transfert de ce fluide et, agencé à l'extérieur dudit conduit, des moyens de détection (6a) de ces particules, lesquelles sont atténuées par une paroi dudit conduit et/ou par ce fluide, ce dispositif comperant moins un tronçon de comptage (4a) et lesdits moyens de détection s'étendant transversalement à ce tronçon en regard de toute sa largeur interne et de part et d'autre de celle-ci, ce dispositif étant **caractérisé en ce que** ledit tronçon de comptage a une section transversale oblongue qui relie entre elles deux portions adjacentes de ce conduit présentant une plus grande section de passage et qui présente un ratio [hauteur interne (h) / largeur interne (l)] inférieur ou égal à 20 %, où la hauteur et la largeur internes représentent respectivement la plus petite et la plus grande des dimensions transversales de ce tronçon mesurées selon deux directions sensiblement perpendiculaires.

2. Dispositif de comptage (6) de particules selon la revendication 1, **caractérisé en ce que** ledit fluide est un liquide et **en ce que** ledit tronçon de comptage (4a) présente un ratio [hauteur interne (h) / largeur interne l)] compris entre 5 % et 10 %.

3. Dispositif de comptage (6) de particules selon la revendication 1 ou 2, **caractérisé en ce que** le ratio de la section de passage du tronçon (4a) sur celle de chaque portion adjacente est inférieur ou égal à 35%.

4. Dispositif de comptage (6) de particules selon la revendication 3, **caractérisé en ce que** ladite hauteur interne (h) du tronçon de comptage (4a) est inférieure à 20 % du diamètre interne de chacune des portions cylindriques adjacentes, et **en ce que** ladite largeur interne l) de ce tronçon (4a) est supérieure à 1,3 fois ce diamètre interne.

5. Dispositif de comptage (6) de particules selon une des revendications précédentes, **caractérisé en ce que** ce tronçon de comptage (4a) présente une section transversale sensiblement rectangulaire dont les grands côtés et/ou les petits côtés sont incurvés selon des courbures symétriques l'une de l'autre, de sorte que ce tronçon présente au moins en partie une face externe sensiblement convexe ou concave.

6. Dispositif de comptage (6) de particules selon la revendication 2, **caractérisé en ce que** ledit conduit est adapté à la circulation de microéchantillons liquides, tels que des microéchantillons sanguins, ladite hauteur interne (h) du tronçon de comptage (4a) étant comprise entre 100 µm et 250 µm et ladite largeur interne (l) de ce tronçon étant supérieure à 1,3 mm, alors que la ou chaque portion cylindrique adjacente présente un diamètre compris entre 0,8 mm et 1,2 mm.

7. Dispositif de comptage (6) de particules selon la revendication 6, **caractérisé en ce que** l'aire de la section de passage dudit tronçon de comptage (4a) est comprise entre 0,15 mm² et 0,25 mm², et **en ce que** ce tronçon présente une longueur comprise entre 30 mm et 40 mm de sorte à pouvoir contenir un microéchantillon d'environ 8 µL en regard desdits moyens de détection.

8. Dispositif de comptage (6) de particules selon une des revendications précédentes, **caractérisé en ce que** ledit tronçon de comptage (4a) présente une paroi d'épaisseur e, exprimée en µm, et de masse volumique d, exprimée en g/cm³, dont le produit e.d est inférieur à 100, de telle sorte que l'atténuation par ce tronçon des particules à compter soit minimisée lorsque ces particules sont des électrons ou des positons issus de rayonnements radioactifs bêta émis par ledit fluide.

9. Dispositif de comptage (6) de particules selon la revendication 8, **caractérisé en ce que** ce produit e.d est inférieur à 50.

10. Dispositif de comptage (6) de particules selon la revendication 8 ou 9, **caractérisé en ce que** ledit tronçon de comptage (4a) est à base d'un polymère thermoformé de masse volumique inférieure ou égale à 1,5 g/cm³, de préférence un polyimide de dénomination « Kapton », et **en ce que** ce tronçon présente une paroi d'épaisseur inférieure à 50 µm et de préférence inférieure à 30 µm.

11. Dispositif de comptage (6) de particules selon une des revendications précédentes, **caractérisé en ce que** lesdits moyens de détection comportent deux ensembles de détecteurs (6a) respectivement disposés contre ou à proximité immédiate de deux grandes faces (4aa) sensiblement planes dudit tronçon de comptage (4a) qui sont distantes entre elles de ladite hauteur (h) et qui sont reliées entre elles par deux petites faces (4ab) de ce tronçon, lesquelles sont dépassées dans le sens de ladite largeur (l) par ces ensembles de détecteurs.

12. Dispositif de comptage (6) de particules selon la revendication 11, **caractérisé en ce qu'**il est agencé en amont d'une pompe péristaltique (7) pilotée par ordinateur pour l'aspiration par à-coups d'une quantité déterminée d'échantillons à prélever, lesdites grandes faces (4aa) dudit tronçon de comptage (4a) présentant chacune en leur côté externe une forme légèrement convexe.

## Claims

1. A particle counting device (6), for the improved sensitivity counting of elementary particles emitted by a fluid, the device comprising a line for transferring this fluid and, placed outside said line, detection means (6a) for detecting these particles, said particles being attenuated by a wall of said line and/or by this fluid, this device including at least one counting portion (4a) and said detection means extending transversely to this portion, facing its entire internal width and on either side thereof, this device being **characterized in that** said counting portion has an oblong cross section which joins together two adjacent portions of this line having a larger flow section and which has an [internal height (h)/internal width (l)] ratio of 20% or less, in which the internal height and the internal width represent the smallest and largest transverse dimensions respectively of this portion, these being measured along two approximately perpendicular directions.

2. The particle counting device (6) as claimed in claim 1, **characterized in that** said fluid is a liquid and wherein said counting portion (4a) has an [internal height (h)/internal width (l)] ratio of between 5% and 10%.

3. The particle counting device (6) as claimed in claim 1 or 2, **characterized in that** the ratio of the flow section of the counting portion (4a) to that of each adjacent portion is equal to or less than 35%.

4. The particle counting device (6) as claimed in claim 3, **characterized in that** said internal height (h) of the counting portion (4a) is less than 20% of the internal diameter of each of the adjacent cylindrical portions and wherein said internal width (l) of this portion (4a) is greater than 1.3 times this internal diameter.

5. The particle counting device (6) as claimed in one of the preceding claims, **characterized in that** this counting portion (4a) has an approximately rectangular cross section, the long sides and/or the short sides of which are curved with mutually symmetrical curvatures, so that this portion has at least partly a substantially convex or concave external face.

6. The particle counting device (6) as claimed in claim 2, **characterized in that** said line is designed for the flow of liquid microsamples, such as blood microsamples, said internal height (h) of the counting portion (4a) being between 100 µm and 250 µm and said internal width (l) of this portion being greater than 1.3 mm, whereas the or each adjacent cylindrical portion has a diameter of between 0.8 mm and 1.2 mm.

7. The particle counting device (6) as claimed in claim 6, **characterized in that** the area of the flow section of said counting portion (4a) is between 0.15 mm² and 0.25 mm² and wherein this portion has a length of between 30 mm and 40 mm so as to be able to contain a microsample of about 8 µl facing said detection means.

8. The particle counting device (6) as claimed in one of the preceding claims, **characterized in that** said counting portion (4a) has a wall thickness e, expressed in µm, and a density d, expressed in g/cm³, the product e x d of which is less than 100, in such a way that the attenuation by this portion of particles to be counted is minimized when these particles are electrons or positrons coming from beta-radioactive radiation emitted by said fluid.

9. The particle counting device (6) as claimed in claim 8, **characterized in that** this product e x d is less than 50.

10. The particle counting device (6) as claimed in claim 8 or 9, **characterized in that** said counting portion (4a) is based on a thermoformed polymer with a density of 1.5 g/cm³ or less, preferably a polyimide of the "Kapton" brand, and wherein this portion has a wall thickness of less than 50 µm and preferably less than 30 µm.

11. The particle counting device (6) as claimed in one of the preceding claims, **characterized in that** said detection means comprise two sets of detectors (6a) placed respectively against or in the immediate vicinity of two approximately plane large faces (4aa) of said counting portion (4a), which faces are separated from each other by said height (h) and are joined together by two small faces (4ab) of this portion, these sets of detectors overhanging said small faces in the direction of said width (l).

12. The particle counting device (6) as claimed in claim 11, **characterized in that** it is placed upstream of a computer-controlled peristaltic pump (7) for sucking up, in bursts, a specified amount of samples to be taken, said large faces (4aa) of said counting portion (4a) each having a slightly convex shape on their external side.

## Patentansprüche

1. Vorrichtung (6) zur Zählung von durch ein Fluid emittierenden Elementarteilchen mit einer verbesserten Empfindlichkeit, wobei die Vorrichtung eine Leitung zum Transfer dieses Fluids und, am äußeren dieser Leitung angeordnet, Mittel zur Erfassung (6a) dieser Teilchen, welche durch eine Wand dieser Leitung und/oder durch dieses Fluid abgeschwächt sind, aufweist, wobei diese Vorrichtung wenigstens einen Zählungsabschnitt (4a) umfasst und die Mittel zur Erfassung sich quer zu diesem Abschnitt gegenüber seiner gesamten inneren Breite und auf beiden Seiten davon erstrecken, wobei diese Vorrichtung **dadurch gekennzeichnet ist, dass** der Abschnitt zur Zählung einen länglichen Querschnitt aufweisen, welcher zwei benachbarte Abschnitte dieser Leitung untereinander verbindet, welche einen größeren Durchgangsquerschnitt aufweist, und welcher ein Verhältnis [innere Höhe (h)/innere Breite (1)] aufweist, welches kleiner oder gleich 20% ist, wobei die innere Höhe und Breite die kleinste bzw. die größte der transversalen Abmessung dieses Abschnitts darstellen, welche entlang von zwei im Wesentlichen senkrechten Richtungen gemessen sind.

2. Vorrichtung (6) zur Zählung von Teilchen nach Anspruch 1, **dadurch gekennzeichnet, dass** das Fluid eine Flüssigkeit ist und dass der Zählungsabschnitt (4a) ein Verhältnis [innere Höhe (h)/innere Breite (1)] aufweist, welches zwischen 5% und 10% enthalten ist.

3. Vorrichtung (6) zur Zählung von Teilchen nach Anspruch 1 oder 2, **dadurch gekennzeichnet, dass** das Verhältnis des Durchgangsquerschnitts des Abschnitts (4a) zu demjenigen von jedem benachbarten Abschnitt kleiner oder gleich 35% ist.

4. Vorrichtung (6) zur Zählung von Teilchen nach Anspruch 3, **dadurch gekennzeichnet, dass** die innere Höhe (h) des Zählungsabschnitts (4a) kleiner als 20% des inneren Durchmessers von jedem der benachbarten zylindrischen Abschnitte ist, und dass die innere Breite (1) dieses Abschnitts (4a) größer als 1,3 mal dieser innere Durchmesser ist.

5. Vorrichtung (6) zur Zählung von Teilchen nach einem der vorhergehenden Ansprüche, **dadurch gekennzeichnet, dass** dieser Zählungsabschnitt (4a) einen im Wesentlichen rechteckigen Querschnitt aufweist, von welchem die langen Seiten und/oder die kurzen Seiten gemäß zueinander symmetrischen Krümmungen gekrümmt sind, so dass dieser Abschnitt wenigstens teilweise eine im Wesentlichen konvexe oder konkave Außenseite aufweist.

6. Vorrichtung (6) zur Zählung von Teilchen nach Anspruch 2, **dadurch gekennzeichnet, dass** die Leitung ausgestaltet ist zur Zirkulation von flüssigen Mikroproben, wie zum Beispiel Blutmikroproben, wobei die innere Höhe (h) des Zählungsabschnitts (4a) zwischen 100 µm und 250 µm enthalten ist und die innere Breite (1) dieses Abschnitts größer als 1,3 mm ist, sowie dass der oder jeder benachbarte zylindrische Abschnitt einen Durchmesser aufweist, welcher zwischen 0,8 mm und 1,2 mm enthalten ist.

7. Vorrichtung (6) zur Zählung von Teilchen nach Anspruch 6, **dadurch gekennzeichnet, dass** die Fläche des Durchgangsquerschnitts des Zählungsabschnitts (4a) zwischen 0,15 mm² und 0,25 mm² enthalten ist, und dass dieser Abschnitt eine Länge aufweist, welche zwischen 30 mm und 40 mm enthalten ist, so dass er eine Mikroprobe im Bereich von 8 µL gegenüber den Detektionsmitteln enthalten kann.

8. Vorrichtung (6) zur Zählung von Teilchen nach einem der vorhergehenden Ansprüche, **dadurch gekennzeichnet, dass** der Zählungsabschnitt (4a) eine Wand mit Stärke e, ausgedrückt in µm, und Dichte d, ausgedrückt in g/cm³, aufweist, deren Produkt e.d kleiner als 100 ist, so dass die Abschwächung der zu zählenden Teilchen durch diesen Abschnitt minimiert wird, wenn die Teilchen Elektronen oder Positronen aus von dem Fluid emittierten radioaktiven Betastrahlen sind.

9. Vorrichtung (6) zur Zählung von Teilchen nach Anspruch 8, **dadurch gekennzeichnet, dass** das Produkt e.d kleiner als 50 ist.

10. Vorrichtung (6) zur Zählung von Teilchen nach Anspruch 8 oder 9, **dadurch gekennzeichnet, dass** der Zählungsabschnitt (4a) auf Basis eines thermogeformten Polymers mit einer Dichte, welche kleiner oder gleich 1,5 g/cm³ ist, vorzugsweise eines Polyimids mit Bezeichnung "Kapton" ist, und dass dieser Abschnitt eine Wandstärke von weniger als 50 µm und bevorzugt weniger als 30 µm aufweist.

11. Vorrichtung (6) zur Zählung von Teilchen nach einem der vorhergehenden Ansprüche, **dadurch gekennzeichnet, dass** die Detektionsmittel zwei Ensembles von Detektoren (6a) aufweisen, welche entsprechend an oder in unmittelbarer Nähe der im Wesentlichen ebenen großen Seiten (4aa) des Zählungsabschnitts (4a) angeordnet sind, welche voneinander um die Höhe (h) beabstandet sind und untereinander durch zwei kurze Seiten (4ab) dieses Abschnitts verbunden sind, welche in der Richtung der Breite (1) durch diese Ensembles von Detektoren überragt sind.

12. Vorrichtung (6) zur Zählung von Teilchen nach Anspruch 11, **dadurch gekennzeichnet, dass** sie vorgelagert einer Peristaltikpumpe (7) angeordnet ist, welche rechnergesteuert zum stoßweise Ansaugen einer vorbestimmten Menge von zu entnehmenden Proben ist, wobei die großen Seiten (4aa) des Zählungsabschnitts (4a) jeweils an ihrer Außenseite eine leicht konvexe Form aufweisen.
